# EUROPÄISCHE PATENTSCHRIFT

(11) **EP 2 473 421 B1**
(45) Veröffentlichungstag und Bekanntmachung des Hinweises auf die Patenterteilung: **15.06.2016**
(21) Anmeldenummer: 10739855.4
(22) Anmeldetag: 19.07.2010
(51) Int. Cl.: B65D 75/32, B65D 75/36, E03D 9/02, A61L 9/05

(54) **Verpackungseinheit aus Applikator und stückförmiges Mittel für den Sanitärbereich**
Packaging unit comprising a chemical agent and an applicator for the bar-shaped agent
Unité d'emballage comprenant un produit sanitaire et un applicateur pour celui

(30) Priorität: 02.09.2009 DE 102009039675
(43) Veröffentlichungstag der Anmeldung: 11.07.2012
(73) Patentinhaber: Buck-Chemie GmbH, 71083 Herrenberg (DE)
(72) Erfinder: JAESCHKE, Edgar, 70794 Filderstadt (DE); LEIPOLD, Joachim, 72760 Reutlingen (DE); KAUTH, Simon, 2116 Mössingen (DE); FRITZ, Matthias, 72810 Gomaringen (DE)
(74) Vertreter: Mammel, Ulrike
(86) Internationale Anmeldenummer: PCT/EP2010/004387
(87) Internationale Veröffentlichungsnummer: WO 2011/026539

(56) Entgegenhaltungen:
- WO-A1-01/64543
- WO-A1-2008/125425
- DE-A1- 10 222 009
- FR-A- 1 210 323
- FR-A1- 2 813 384
- US-A1- 2007 007 302

## Beschreibung

Die vorliegende Erfindung betrifft eine Verpackungseinheit umfassend ein stückförmiges Mittel für den Sanitärbereich und eine Vorrichtung zur Applikation und die Verwendung derselben.

Zur Vermeidung der vom Verbraucher als unhygienisch empfundenen WC-Körbchen sind aus der WO 99/66017 haftende pastöse Mittel bekannt, die direkt auf die Oberfläche des Sanitärgegenstands aufgebracht werden, dort haften und erst nach einer größeren Anzahl von Spülungen abgespült werden.

Eine Applikationsvorrichtung für fließfähige Tollettenreinigungsmittel in Form einer Spritze ist aus der US 2007/0007302 A1 und eine Blisterverpackung zum Einmalgebrauch ist aus der WO 01/64543 A1 bekannt. Aus der DE 102 22 009 A1 ist eine Vorrichtung zur Aufnahme eines in das Innere eines Toilettenbeckens abzugebenden eiförmigen Zubereitung bekannt.

Die Anmelderin hat zwischenzeitlich auch stückförmigen feste Mittel für den Sanitärbereich wie beispielsweise Rimblocks mit einer oder mehr Phasen, Rimblocks mit Duftphase, Rimblocks mit Bleichmittelphase,

Beduftungstabletten, Entkalkungstabletten, Reinigungstabletten, Mittel aus Kunststoff etc., die mittels eines Haftmittels direkt an der Toilettenoberfläche appliziert werden, entwickelt. Diese Sanitärmittel werden vom Spülstrom, wie auch das die Befestigung bewirkende Haftmittel, nach und nach abgespült, wie in der älteren Anmeldung PCT/EP2009/000873 beschrieben ist. Die Aufgabe der Erfindung besteht darin, eine Vorrichtung anzugeben, die dem Verbraucher eine einfache, kostengünstige und hygienische Applikation dieser Sanitärmittel direkt auf die Oberfläche In der Toilettenschüssel ermöglicht. Weiterhin besteht die Aufgabe darin, eine Verpackungseinheit bereitzustellen. Diese Aufgabe wird durch die Merkmale des Anspruchs 1 gelöst.

Die erfindungsgemäße Vorrichtung, die zur Applikation von stückförmigen Sanitärmitteln dient, umfasst einen Hohlraum, in dem das Sanitärmittel aufgenommen wird. Weiterhin sind Befestigungsmittel vorgesehen, die der Befestigung des Sanitärmittels in dem Hohlraum dienen sowie Ausgabeelemente, die dazu dienen, das Sanitärmittel bei deren Betätigung wieder aus der Vorrichtung freizugeben.

Die Ausgabeelemente befinden sich oberhalb des Hohlraums, in dem das Sanitärmittel aufgenommen wird, sind höckerförmig ausbildet und durch eine V-förmige Nut voneinander getrennt. Die Ausgabeelemente weisen zwei Angriffsflächen auf, durch deren Gegeneinanderdrücken die Befestigungsmittel lösbar sind.

Diese Vorrichtung ermöglicht eine berührungslose Applikation des mit einem Klebemittel beschichteten Mittels auf der Oberfläche der Toilettenschüssel. Die Hand des Verbrauchers kommt ausschließlich mit dem Applikator in Kontakt, ein jeglicher Kontakt mit der Tollettenschüssel und dem klebenden Mittel wird vermieden.

Weiterhin ermöglicht die erfindungsgemäße Vorrichtung die einhändige Applikation des Mittels. Auch ein Hineingreifen mit zwei Händen in den Bereich der Toilettenschüssel wird somit vermieden.

Ein weiterer Vorteil der Erfindung besteht darin, dass die Applikationsvorrichtung gleichzeitig einen Handschutz darstellt und so ein jeder Kontakt des Verbrauchers mit dem Mittel selbst und der Toilette vermieden wird.

Auch ist die erfindungsgemäße Vorrichtung einfach und kostengünstig herstellbar.

Ein wesentlicher Vorteil der erfindungsgemäßen Vorrichtung besteht darin, dass sie nicht nur zur Applikation des Mittels, insbesondere zur berührungslosen Einhandapplikation, dient, sondern dass sie gleichzeitig auch als Teil der Sichtverpackung des Mittels (Blister) dienen kann.

Die Befestigung des Mittels in der Vorrichtung kann verschiedenartig, beispielsweise durch Reibung, Pressen, Kleben und/oder Untergreifen, erfolgen. Vorzugsweise sind die Befestigungsmittel an den senkrecht (in Z-Richtung) verlaufenden Wänden des Hohlkörpers angeordnet, und die Befestigung des Sanitärmittels erfolgt durch Reibung/Flächenpressung. Im Falle der Befestigung über Flächenpressung/Reibung sind wenigstens zwei, vorzugsweise wenigstens drei und besonders bevorzugt wenigstens vier Anlageflächen für die Flächenpressung erforderlich.

Befestigungsmittel im Sinne der vorliegenden Erfindung sind Mittel, die ein selbständiges Halten des Sanitärmittels in dem Hohlraum auch dann bewirken, wenn der Behälter mit der Öffnung nach unten gerichtet ist. Zusätzlich zu einer "drucklosen" Berührung von Hohlkörperwand und Sanitärmittel müssen noch weitere Mittel vorgesehen sein, die entweder das Sanitärmittel gegen die Hohlkörperwand pressen bzw. eine Klebeverbindung vorsehen, oder aber das Sanitärmittel untergreifen und so ein Herausfallen verhindern.

Keine Befestigungsmittel im Sinne vorliegenden Erfindung liegen vor, wenn das in dem Hohlraum befindliche Mittel bei einem nach unten offenen Hohlraum ohne Betätigung der Ausgabeelemente herausgleiten würde, wie es beispielsweise bei den aus der FR 2 813 384 A1 oder der FR 1 210 323 bekannten Eiswürfelbehältern der Fall ist, die zudem nur bei Temperaturen unter 0°C und nicht wie der erfindungsgemäße Behälter bei Raumtemperatur einsetzbar ist.

Bei einem quaderförmigen Sanitärmittel sind die klemmenden Befestigungsmittel vorzugsweise derart angeordnet, dass sie ebenfalls auf der Außenfläche eines gedachten Quaders liegen und die aus von dem Eiswürfelbehälter bekannte konische oder pyramidal verlaufenden Seitenwände, die ein Herausgleiten fördern, gerade nicht aufweisen.

Bezüglich der Ausgabeelemente wird unter "höckerförmig" ein jedes hervorstehendes Formen verstanden, sei es eher quadratisch, trapezförmig, keilförmig, konisch oder anderweitig geformt. Durch das Merkmal des "Hervorstehens" ergibt sich gleichzeitig auch, dass seitlich Flächen bestehen, die als Angriffsflächen dienen können. Dadurch, dass die beiden Ausgabeelemente durch eine V-Nut voneinander getrennt sind, wird die gewünschte Knickbewegung ermöglicht.

Durch die erfindungsgemäße Befestigung des Mittels in der Vorrichtung derart, dass die Klebemittelschicht des Mittels einen geringfügigen Abstand B zu der Fläche aufweist, auf der die Vorrichtung auf ihrem Rand aufgestellt oder befestigt ist, ist die Klebemittelschicht durch die umgebende Vorrichtung und den geringfügigen Abstand B, beispielsweise gegenüber der Blisterkarte, vor Beschädigungen geschützt. Die im Stand der Technik häufig anzutreffende zusätzliche Schutzfolie für solche Klebeschichten, beispielsweise aus Papier, Polyethylen, Polypropylen, silikonierten oder fluorierten Folien, die gleichzeitig jedoch ein unerwünschtes Abtragen der Klebemittelschicht und damit u.U. ein schlechtes Kleben mit sich bringen kann, kann somit entfallen.

Der Abstand B beträgt im Allgemeinen zwischen 1 mm und 10 mm, bevorzugt zwischen 2 mm und 5 mm und besonders bevorzugt zwischen 1,5 mm und 3 mm.

Die erfindungsgemäße Vorrichtung kann zur Applikation und Verpackung von quaderförmigen, scheibenförmigen oder auch von anders geformten dreidimensionalen stückförmigen Sanitärmitteln verwendet werden. Im Allgemeinen weisen alle stückförmigen Sanitärmittel wenigstens eine ebene Fläche auf, die die Klebemittelschicht trägt und mittels der das Mittel in der Toilettenschüssel befestigt werden kann. Bezüglich der Gestaltung der anderen Seiten und der Form des Mittels bestehen jedoch keine prinzipiellen Beschränkungen. Soll beispielsweise ein Applikator für ein scheibenförmiges Mittel bereitgestellt werden, so sind der Hohlraum, der das Sanitärmittel aufnimmt, und die Befestigungsmittel an die jeweilige Form des Sanitärmittels anzupassen.

In der bevorzugten Ausführungsform ist das in der Vorrichtung befindliche Mittel derart in der Vorrichtung befestigt, dass es aus keiner Raumposition aus der Vorrichtung ohne Abdeckung durch eine Blisterrückwand herausfallen kann, d.h. die Vorrichtung ist bezüglich des Mittels selbsthaltend ausgebildet.

Die Erfindung wird nachfolgend anhand von Ausführungsbeispielen näher beschrieben. Es zeigen:
- Figur 1: eine perspektivische Ansicht des Applikators,
- Figur 2: eine schematische Ansicht der Längsseite des Applikators mit einem Sanitärmittel,
- Figur 3: eine schematische Ansicht der Längsseite des Applikators mit einem Sanitärmittel nach Betätigung der Ausgabeelemente,
- Figur 4: eine Ansicht der Längsseite des Applikators ohne Sanitärmittel,
- Figur 5: einen Längsschnitt durch die Vorrichtung aus Figur 1 entlang der Linie A-A mit einem Sanitärmittel,
- Figur 6: eine Draufsicht auf die Schmalseite der Vorrichtung,
- Figur 7: eine Draufsicht auf die Vorrichtung von unten (XY-Ebene),
- Figur 8: eine Ansicht der aus dem Applikator, dem Mittel und der Blisterrückwand bestehenden Verpackungseinheit bei vertikaler Ausrichtung des Applikators,
- Figur 9: eine Ansicht der aus dem Applikator, dem Mittel und der Blisterrückwand bestehenden Verpackungseinheit bei horizontaler Ausrichtung des Applikators und
- Figur 10: eine perspektivische Ansicht einer alternativen Ausführungsform mit drei Befestigungsmitteln in der Seitenwand.

Der Applikator 11 ist ein Hohlkörper, der einen Hohlraum 22 zur Aufnahme des stückförmigen festen Sanitärmittels 13 aufweist. Er weist an der Unterseite des Applikators eine Öffnung 21 auf, durch die das Sanitärmittel 13 in den Hohlraum 22 eingefügt werden kann.

Weiterhin umfasst der Applikator Befestigungsmittel 15, 16, 17, 18, die dazu dienen, das Sanitärmittel 13 sicher zu halten und Ausgabeelemente 24, 25 an der Oberseite des Applikators 11, die dazu dienen, das Sanitärmittel 13 wieder freizugeben.

Der Applikator 11 dient sowohl zur berührungslosen Applikation eines mit einem Klebemittel 14 beschichteten Sanitärmittels 13 auf einen Sanitärgegenstand als auch als Verpackung des Mittels. Gleichzeitig dient er auch als Handschutz bei der Applikation.

Die sichere Befestigung des Sanitärmittels 13 in dem Applikator 11 ist erforderlich, um zu verhindern, dass das mit einem Klebemittel 14 beschichtete Sanitärmittel 13 unbeabsichtigt in den dargestellten Figuren "nach unten" in Z-Richtung rutscht oder gar aus der Öffnung 21 heraus und an der Blisterrückwand anklebt.

Dieser Schutz der Klebemittelfläche 14 durch den Abstand B bewirkt, dass auf eine zusätzliche Schutzfolie für die Klebemittelschicht 14 verzichtet werden kann.

Verschiedene Mittel 15, 16, 17, 18 sind zur klemmenden Befestigung in Form von an der Hohlkörperwand in Richtung des Sanitärmittels 13 hervorspringende Flächen oder Nasen vorgesehen, die das Mittel 13 über Reibung/Flächenpressung sicher halten. Die nach innen weisenden Flächen der Befestigungsmittel 15, 16, 17, 18 liegen auf den Außenflächen eines gedachten Quaders und die Außenflächen des gedachten Quaders verlaufen parallel der z-Achse. Die nach innen weisenden Flächen der Befestigungsmittel 15, 16, 17, 18 bilden somit im Gegensatz zu dem Eiswürfelbehälter keinen Konus.

Alternativ kann das Mittel 13 auch in dem Applikator durch Vorsprünge oder Nasen oder Verankerungsmittel gehalten werden oder in dem Applikator angeklebt oder auf sonstige Weise befestigt sein. Selbstverständlich muss eine jede Befestigung so gewählt werden, dass das Mittel 13 bei Betätigung der Ausgabeelemente 24, 25 aus der Befestigung freigegeben werden kann.

Bei der vorliegenden Ausführungsform befinden sich die Befestigungsmittel 15, 16, 17, 18 an den im Wesentlichen senkrecht (Z-Achse) verlaufenden Wänden 20, 27 des Hohlkörpers im Bereich des Mittels 13. Jeweils ein Befestigungselement 15, 16 ist durch die Innenseite einer nahe der Schmalseite 26 der Vorrichtung annähernd senkrecht (in Z-Richtung) zum Rand 19 verlaufenden Wand 20 eines nach innen weisenden Vorsprungs 30, 31 gebildet. Die Wand 20 kann nach oben geringfügig in Richtung der Mittelebene M (in YZ-Richtung) geneigt sein, um einen festen und wieder lösbaren Sitz zu gewährleisten.

Weiterhin sind zwei Befestigungselemente 17, 18 in der Seitenwand 27 an den Längsseiten 28 des Applikators vorgesehen, die sich symmetrisch der Mittelebene M erstrecken und diese durchqueren sowie in Form von nach innen hervorstehenden Nasen ausgebildet sind.

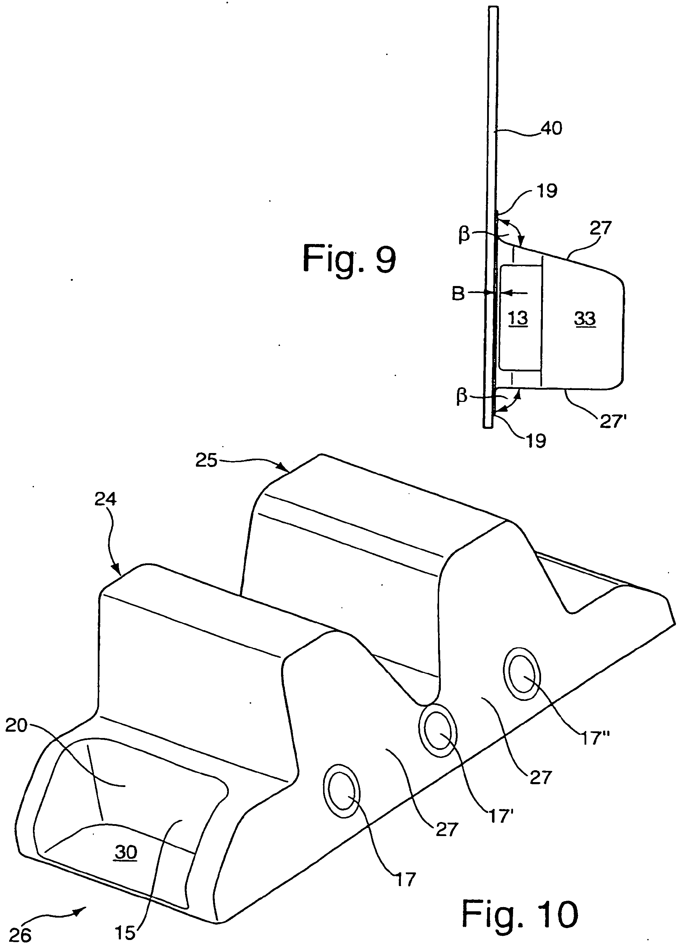

In der alternativen Ausführungsform gemäß Figur 10 weist der Applikator in jeder Wand 20 der Schmalseite 26 Befestigungsmittel 15, 16 und in der nahezu senkrecht verlaufenden, geringfügig konisch nach oben zulaufenden Wand 27 an der Längsseite 28 drei Befestigungsmittel 17, 17', 17" in Form von nach innen weisenden Nasen auf.

Oberhalb des Hohlraums 22 für das Mittel 13 umfasst der Applikator 11 zwei Ausgabeelemente 24, 25, die dazu dienen, den mittels der Befestigungsmittel 15, 16, 17, 18 transportsicher befestigten Block 13 freizugeben, so dass das Mittel gegen die Toilettenoberfläche gedrückt werden kann.

Die Ausgabeelemente 24, 25 sind höckerförmig ausgebildet und durch eine V-Nut 32 voneinander getrennt. Der Winkel α zwischen den beiden die V-Nut begrenzenden Flächen 35, 36 ist variabel und beträgt vorzugsweise zwischen 30° und 70°.

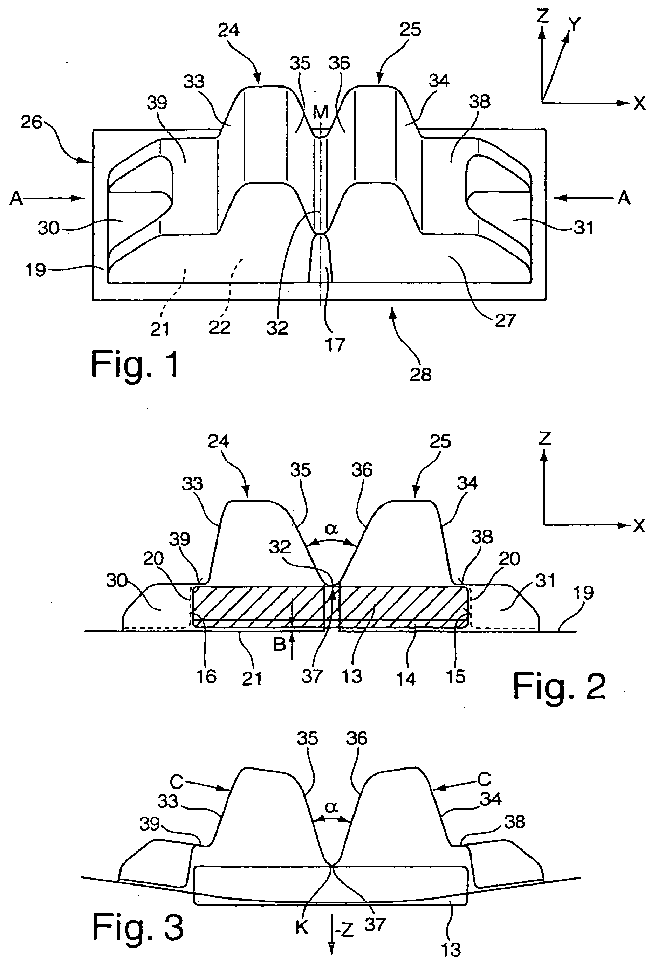

Die der V-Nut 32 entgegengesetzten Seiten der Ausgabeelemente 24, 25 weisen diese zwei Angriffsflächen 33, 34 auf. Greift man von oben - beispielsweise mit dem Daumen und Zeigefinger einer Hand - auf die beiden Angriffsflächen 33, 34 und drückt diese etwas gegeneinander etwa in Form einer "Knick-Bewegung" - (siehe Pfeil C in Figur 3), so wird der Winkel α zwischen den Flächen 35, 36 verringert, der den rechten Teil des Hohlraums 22 begrenzende Teil des Applikators bewegt sich etwa auf einer Kreisbahn um die Knicklinie K in der V-Nut 32 nach rechts oben und der den linken Teil des Hohlraums 22 begrenzende Teil des Applikators nach links oben (vgl. Figuren 2, 3).

Durch diese Knickbewegung auf die beiden Flächen 33, 34 werden die Befestigungselemente 15, 16, 17, 18 gelöst und das Mittel 13 freigegeben.

Für eine bessere Betätigbarkeit können die beiden Betätigungsflächen 33, 34 auch Riffelungen oder Griffmulden aufweisen.

Der Wandabschnitt 37 an der Unterseite der V-Nut 32 und die beiden sich seitlich an die Angriffsflächen 33, 34 horizontal anschließenden Flächen 38, 39 bilden im Ruhezustand mit ihren Innenflächen eine das Mittel nach oben begrenzende horizontale Anlagefläche (vgl. Fig. 2).

Durch die Knickbewegung werden die beiden seitlichen Flächen 38, 39 weggeschwenkt, das freigegebene Mittel stützt sich nur noch auf dem Wandabschnitt 37 ab und kann durch eine zusammen mit der Knickbewegung gleichzeitig erfolgenden Vorwärtsbewegung in -Z-Richtung nun unter Abstützung gegen den Wandabschnitt 37 gegen die Oberfläche des Sanitärgegenstands gedrückt und dadurch angeklebt werden. Der Wandabschnitt 37 bildet die Vorschubfläche für die Ausgabe des Mittels.

Um ein einfaches und reproduzierbares Knicken zu gewährleisten, weist die Vorrichtung beidseitig Soll-Knickstellen in der Mittelebene M in der seitlichen Applikatorwand 27 auf, wobei es bevorzugt ist, die Befestigungsmittel 17, 18 in Kombination mit der V-Nut 32 gleichzeitig als Sollknickstelle auszubilden.

Die bei der Knickbewegung aufzuwendende Kraft hängt von der Erstreckung der Ausgabeelemente in Z-Richtung, vom Winkel α und von dem Abstand der Betätigungsflächen 33, 34 von der Mittelebene M ab.

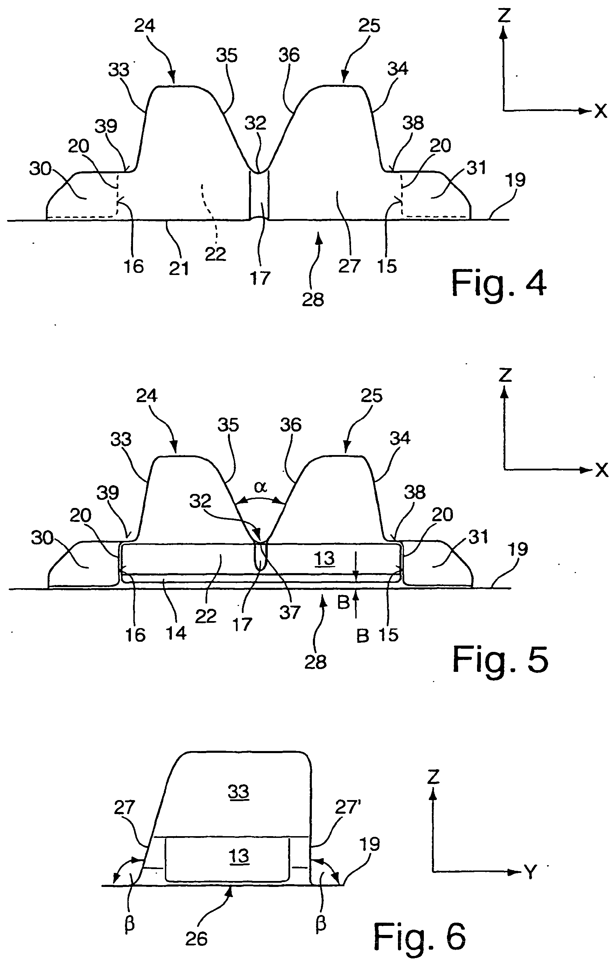

Das Mittel 13 ist in dem Applikator so befestigt, dass die Klebemittelschicht 14 des Mittels 13 geringfügig oberhalb der Unterseite des horizontal umlaufenden Randes 19 der Applikators endet und somit immer ein Abstand B zwischen dem unteren Ende des klebenden Sanitärmittels 13 und der Unterseite des Randes 19 beziehungsweise der Aufstellfläche besteht (vgl. Figuren 2 und 5), so dass die Klebemittelschicht 14 eines in dem Applikator befindlichen Mittels, das mit seinem Rand 19 auf eine Fläche gestellt ist, auf jeden Fall die Fläche nicht berührt. Der in Z-Richtung gemessene Abstand B zwischen der Unterseite des Randes 19 beziehungsweise Auflagefläche und dem Wandabschnitt 37 muss somit größer sein als die Höhe des Mittels 13 nebst Klebemittelschicht 14 in Z-Richtung.

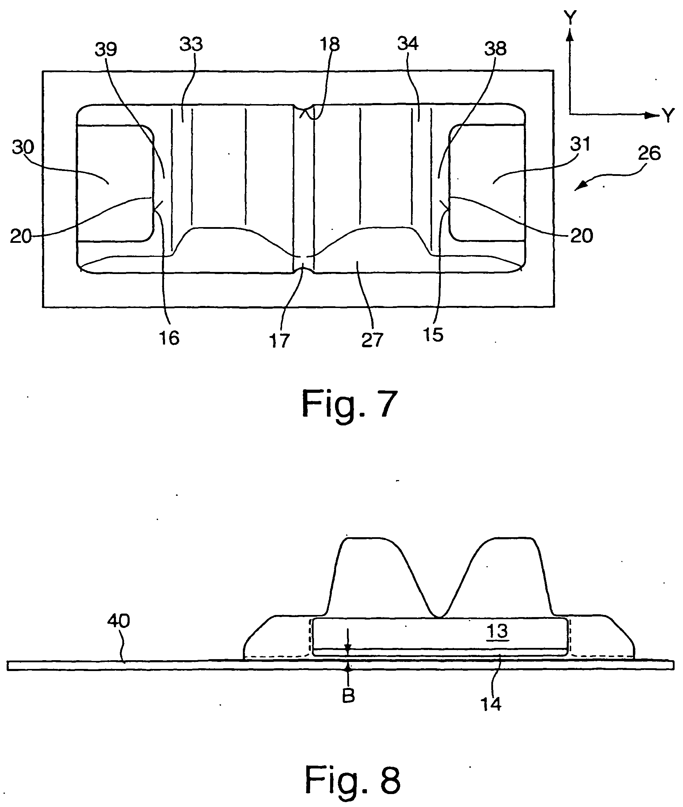

Der Applikator weist weiterhin an seinem unteren Ende einen horizontal in XY-Richtung umlaufenden Rand 19 auf, der zur Fixierung der Vorrichtung nebst dem darin befindlichen Mittel 13 auf einer Blisterkarte 40 oder Rückwand dient, die auf dem nach unten weisenden Rand aufgeklebt oder anderweitig befestigt ist (vgl. Figuren 8 und 9).

Die Neigung der Seitenwände 27, 27' gegenüber dem Rand 19 ist üblicherweise gleich und beträgt zwischen 80° und 110°.

Für einen besseren Stand des Mittels in der Blisterverpackung kann die Neigung der beiden Seitenwände 27, 27' gegenüber dem Rand 19 (XY-Ebene) auch geringfügig unterschiedlich sein (vgl. Figuren 6, 9), so dass bei dem in der Blisterverpackung verpackten Mitteln 13 die nach unten weisende Seite 27' der Verpackung beziehungsweise des Applikators gegenüber der Blisterkarte 40 und damit gegenüber dem Rand 19 unter einem Winkel β zwischen 70° und 90° verläuft. Hierdurch wird eine höhere Standfestigkeit der Verpackungseinheit im Verkaufsregal gewährleistet (Dreipunktauflage).

Der Applikator ist ein Kunststoffformteil, ein geformter Karton oder ein Stanzteil. Im Falle eines Kunststoffformteils ist die Dicke der Kunststofffolie so zu wählen, dass der Applikator die erforderliche Steifigkeit und Beständigkeit aufweist, um bei einem leichten Zusammendrücken (siehe Pfeil C in Figur 3) die Freigabe des Mittels zu ermöglichen. Im Allgemeinen beträgt die Dicke zwischen 100 und 500 µm. In dieser Ausführungsform wurde als Folienmaterial APET (320 mm) der Firma Sichtpack Hagner verwendet.

Die Figuren 8 und 9 zeigen die erfindungsgemäße Verpackungseinheit, die aus dem Applikator, der gleichzeitig als Halbblister dient, den in dem Applikator befestigten Sanitärmittel 13, das mit einer Klebemittelschicht 14 versehen sein kann und einer Blisterkarte 40 besteht.

Die Figuren 8 und 9 unterscheiden sich durch die Orientierung der Vorrichtung auf der Blisterkarte (Längs- oder Queranordnung). Die Blisterkarte dient dazu, das in dem Applikator/der Verpackung befindliche Mittel und auch die Klebemittelschicht zu schützen sowie auch um die Verpackungseinheit in den Verkaufsstellen durch Aufstellen oder Aufhängen zu präsentieren. Die Blisterrückwand umfasst weiterhin die Bezeichnung des Mittels des Herstellers und auch eine Verwendungsanweisung sowie sonstige der Produktbeschreibung hinzugefügte übliche Angaben.

Auf der Blisterkarte 40 können ein oder mehrere Applikatoren 11 aufgebracht sein. Ebenso ist es möglich, die Verkaufseinheit als Vollblister vorzusehen.

## Patentansprüche

1. Verpackungseinheit umfassend ein stückförmiges Sanitärmittel (13) und eine Vorrichtung zur Applikation des stückförmigen Sanitärmittels (13) auf der Oberfläche einer Toilettenschüssel, welche Vorrichtung einen Hohlraum (22) zur Aufnahme des Sanitärmittels (13) aufweist und Elemente (24, 25) zur Ausgabe des Sanitärmittel (13) aus der Vorrichtung, wobei sich die Ausgabeelemente (24, 25) oberhalb des Hohlraums (22) zur Aufnahme des Sanitärmittels (13) befinden und die Vorrichtung an ihrem unteren Ende einen umlaufenden Rand (19) aufweist
und die Vorrichtung Mittel (15, 16, 17, 18) zum Befestigen des Sanitärmittels (13) in dem Hohlraum (22) umfasst,
**dadurch gekennzeichnet,**
**dass** die Ausgabeelemente (24, 25) höckerförmig ausgebindet und durch eine V-förmige Nut voneinander getrennt sind und zwei Angriffsflächen (33, 34) aufweisen, durch deren Gegeneinanderdrücken die Befestigungsmittel (15, 16, 17, 18) lösbar sind,
und an der Unterseite der V-Nut (32) ein Wandabschnitt (37) vorgesehen ist, der als Vorschubfläche zur Ausgabe des Mittels (13) dient,
und der Wandabschnitt (37) an der Unterseite der V-Nut (32) und die beiden sich seitlich an die Angriffsflächen (33, 34) horizontal anschließenden Flächen (38, 39) im Ruhezustand mit ihren Innenflächen eine das Sanitärmittel (13) nach oben begrenzende horizontale Anlagefläche bilden und durch das Gegeneinanderdrücken der Angriffsflächen (33, 34) die beiden seitlichen Flächen (38, 39) weggeschwenkt werden und das freigegebene Mittel sich nur noch auf dem Wandabschnitt (37) abstützt.

2. Verpackungseinheit nach Anspruch 1, **dadurch gekennzeichnet, dass** die Befestigungsmittel (15, 16, 17, 18) an den im Wesentlichen senkrecht verlaufenden Wänden (20, 27) des Hohlkörpers angeordnet sind und dass die Befestigung durch Reibung/Flächenpressung erfolgt.

3. Verpackungseinheit nach einem der vorangegangenen Ansprüche, **dadurch gekennzeichnet, dass** der Winkel α der die V-Nut (32) begrenzenden Flächen (35, 36) zwischen 30° und 70° beträgt.

4. Verpackungseinheit nach Anspruch 3, **dadurch gekennzeichnet, dass** der Winkel α durch das Gegeneinanderdrücken der Angriffsflächen (33, 34) veränderbar ist.

5. Verpackungseinheit nach einem der vorangegangenen Ansprüche, **dadurch gekennzeichnet, dass** Soll-Knickstellen (17, 18) vorgesehen sind.

6. Verpackungseinheit nach einem der vorangegangenen Ansprüche, **dadurch gekennzeichnet, dass** die Vorrichtung aus einer Kunststofffolie, einem geformten Karton oder einem Stanzteil ist.

7. Verpackungseinheit nach Anspruch 6, **dadurch gekennzeichnet, dass** die Dicke der Kunststofffolie zwischen 100 µm und 500 µm liegt.

8. Verpackungseinheit nach einem der Ansprüche 1 bis 7, **dadurch gekennzeichnet, dass** das Sanitärmittel (13) an seiner Unterseite eine Klebemittelschicht (14) aufweist.

9. Verpackungseinheit nach einem der vorangegangenen Ansprüche, **dadurch gekennzeichnet, dass** das Sanitärmittel (13) unverrutschbar in der Vorrichtung (11) befestigt ist.

10. Verpackungseinheit nach einem der vorangegangenen Ansprüche, **dadurch gekennzeichnet, dass** zwischen der Unterseite des Randes (19) der Vorrichtung und der Unterseite des in der Vorrichtung befestigten Sanitärmittels (13) ein Abstand B ist.

11. Verpackungseinheit nach Anspruch 10, **dadurch gekennzeichnet, dass** der Abstand B zwischen 1 mm und 10 mm und vorzugsweise zwischen 2 mm und 5 mm beträgt.

12. Verpackungseinheit nach Anspruch 11, **dadurch gekennzeichnet, dass** der Abstand B zwischen 1,5 mm und 3 mm beträgt.

13. Verpackungseinheit nach einem der vorangegangenen Ansprüche, **dadurch gekennzeichnet, dass** der Rand (19) der Vorrichtung auf einer Blisterkarte (40) befestigt ist.

14. Verwendung einer Verpackungseinheit nach einem der Ansprüche 1 bis 13 zur Applikation eines Sanitärmittels (13) auf der Oberfläche eines Sanitärgegenstands.

15. Verwendung nach Anspruch 14 zur einhändigen berührungslosen Applikation eines Sanitärmittels auf der Oberfläche eines Sanitärgegenstands.

## Claims

1. Packaging unit comprising a piece-form sanitary agent (13) and a device for applying the piece-form sanitary agent (13) to the surface of a toilet bowl, which device has a cavity (22) for accommodating the sanitary agent (13), and elements (24, 25) for dispensing the sanitary agent (13) from the device, wherein the dispensing elements (24, 25) are located above the cavity (22) for accommodating the sanitary agent (13), and the device has an all-round periphery (19) at its lower end,
and the device comprises means (15, 16, 17, 18) for fastening the sanitary agent (13) in the cavity (22),
**characterized**
**in that** the dispensing elements (24, 25) are of protuberance-like design and are separated from one another by a V-shaped groove and have two engagement surfaces (33, 34), it being possible for the fastening means (15, 16, 17, 18) to be released by virtue of said engagement surfaces being pushed towards one another,
and a wall portion (37), which serves as an advancement surface for dispensing the agent (13), is provided on the underside of the V groove (32), and the wall portion (37) on the underside of the V groove (32) and the two surfaces (38, 39) horizontally adjoining the engagement surfaces (33, 34) in the lateral direction form in the rest state, by way of their inner surfaces, a horizontal abutment surface which delimits the sanitary agent (13) in the upward direction and, by virtue of the engagement surfaces (33, 34) being pushed towards one another, the two lateral surfaces (38, 39) are pivoted away and the freed agent is supported just on the wall portion (37).

2. Packaging unit according to Claim 1, **characterized in that** the fastening means (15, 16, 17, 18) are arranged on the essentially vertically running walls (20, 27) of the hollow body, and **in that** the fastening takes place by friction/surface pressing.

3. Packaging unit according to one of the preceding claims, **characterized in that** the angle α of the surfaces (35, 36) delimiting the V groove (32) is between 30° and 70°.

4. Packaging unit according to Claim 3, **characterized in that** the angle α can be changed by virtue of the engagement surfaces (33, 34) being pushed towards one another.

5. Packaging unit according to one of the preceding claims, **characterized in that** predetermined points of inflection (17, 18) are provided.

6. Packaging unit according to one of the preceding claims, **characterized in that** the device is made of a plastics film, shaped cardboard or a punched part.

7. Packaging unit according to Claim 6, **characterized in that** the thickness of the plastics film is between 100 µm and 500 µm.

8. Packaging unit according to one of Claims 1 to 7, **characterized in that** the sanitary agent (13) has an adhesive layer (14) on its underside.

9. Packaging unit according to one of the preceding claims, **characterized in that** the sanitary agent (13) is fastened in a non-slip manner in the device (11).

10. Packaging unit according to one of the preceding claims, **characterized in that** there is a distance B between the underside of the periphery (19) of the device and the underside of the sanitary agent (13) fastened in the device.

11. Packaging unit according to Claim 10, **characterized in that** the distance B is between 1 mm and 10 mm and preferably between 2 mm and 5 mm.

12. Packaging unit according to Claim 11, **characterized in that** the distance B is between 1.5 mm and 3 mm.

13. Packaging unit according to one of the preceding claims, **characterized in that** the periphery (19) of the device is fastened on a blister card (40).

14. Use of a packaging unit according to one of Claims 1 to 13 for applying a sanitary agent (13) to the surface of a sanitary article.

15. Use according to Claim 14 for applying a sanitary agent to the surface of a sanitary article in a contactless manner using one hand.

## Revendications

1. Unité d'emballage comprenant un produit sanitaire (13) sous forme de morceaux et un dispositif d'application du produit sanitaire (13) sous forme de morceaux sur la surface d'une cuvette de toilette, ledit dispositif comportant un espace creux (22) pour la réception du produit sanitaire (13) et des éléments (24, 25) pour la distribution du produit sanitaire (13) hors du dispositif, les éléments de distribution (24, 25) se trouvant au-dessus de l'espace creux (22) pour recevoir le produit sanitaire (13) et le dispositif comportant au niveau de son extrémité inférieure une bordure (19) périphérique et le dispositif comprenant des moyens (15, 16, 17, 18) pour la fixation du produit sanitaire (13) dans l'espace creux (22), **caractérisée en ce que** :
les éléments de distribution (24 ; 25) prennent une forme de bosse et sont séparés l'un de l'autre par une rainure en V et comportent deux surfaces de préhension (33, 34) à travers lesquelles les moyens de fixation (15, 16, 17, 18) sont amovibles par pression mutuelle ; et
une section de paroi (37) est prévue au niveau du côté inférieur de la rainure en V (32), ladite section servant de surface d'alimentation pour la distribution du moyen (13) ; et
la section de paroi (37) située au niveau du côté inférieur de la rainure en V (32) et les deux surfaces (38, 39) se rejoignant à l'horizontale en côté au niveau des surfaces de préhension (33, 34) forment dans l'état de repos avec ses surfaces intérieures une surface d'installation horizontale délimitant vers le haut le produit sanitaire (13) et faisant basculer, par la pression mutuelle des surfaces de préhension (33, 34), les deux surfaces latérales (38, 39) et le moyen se libérant ne s'appuyant plus encore que sur la section de paroi (37).

2. Unité d'emballage selon la revendication 1, **caractérisée en ce que** les moyens de fixation (15, 16, 17, 18) sont disposés contre les parois (20, 27) du corps creux s'étendant pour l'essentiel verticalement et que la fixation se produit par friction/compression de surfaces.

3. Unité d'emballage selon l'une quelconque des revendications précédentes, **caractérisée en ce que** l'angle α des surfaces (35, 36) délimitant la rainure en V (32) se situe entre 30° et 70°.

4. Unité d'emballage selon la revendication 3, **caractérisée en ce que** l'angle α peut être modifié en exerçant une pression mutuelle sur les surfaces de préhension (33, 34).

5. Unité d'emballage selon l'une quelconque des revendications précédentes, caractérisée en ce des plis rainurés théoriques (17, 18) sont prévus.

6. Unité d'emballage selon l'une quelconque des revendications précédentes, **caractérisée en ce que** le dispositif est réalisé à partir d'un film plastique, d'un carton formé ou d'une pièce poinçonnée.

7. Unité d'emballage selon la revendication 6, **caractérisée en ce que** l'épaisseur du film en plastique se situe entre 100 µm et 500 µm.

8. Unité d'emballage selon l'une quelconque des revendications 1 à 7, **caractérisée en ce que** le produit sanitaire (13) comporte une couche de colle (14) au niveau de son côté inférieur.

9. Unité d'emballage selon l'une quelconque des revendications précédentes, **caractérisée en ce que** le produit sanitaire (13) est fixé de façon à ne pouvoir déraper dans le dispositif (11).

10. Unité d'emballage selon l'une quelconque des revendications précédentes, **caractérisée en ce qu'**une distance B est présente entre le côté inférieur de la bordure (19) du dispositif et le côté inférieur du produit sanitaire (13) fixé dans le dispositif.

11. Unité d'emballage selon la revendication 10, **caractérisée en ce que** la distance B se situe entre 1 mm et 10 mm et de préférence entre 2 mm et 5 mm.

12. Unité d'emballage selon la revendication 11, **caractérisée en ce que** la distance B se situe entre 1,5 mm et 3 mm.

13. Unité d'emballage selon l'une quelconque des revendications précédentes, **caractérisée en ce que** la bordure (19) du dispositif est fixée sur une carte en emballage thermocollé (40).

14. Utilisation d'une unité d'emballage selon l'une quelconque des revendications 1 à 13, pour l'application d'un produit sanitaire (13) sur la surface d'un objet sanitaire.

15. Utilisation selon la revendication 14 d'application sans contact à une main d'un produit sanitaire sur la surface d'un objet sanitaire.
